Europäisches Patentamt

European Patent Office

Office européen des brevets

(11) Publication number: **0 318 193**
**A2**

(12) **EUROPEAN PATENT APPLICATION**

(21) Application number: 88310708.8

(22) Date of filing: 14.11.88

(51) Int. Cl.⁴: **A61L 27/00 , A61L 29/00**

(30) Priority: 25.11.87 US 125421

(43) Date of publication of application:
31.05.89 Bulletin 89/22

(84) Designated Contracting States:
**DE FR GB**

(71) Applicant: **DOW CORNING WRIGHT
CORPORATION**
**P.O. Box 100 5677 Airline Road**
**Arlington Tennessee(US)**

(72) Inventor: **Gauger, John Russell Dean**
**99 Shelley Renee Lane**
**Cordova Tennessee(US)**
Inventor: **Jakubczak, Eugene Robert**
**2839 Davies Plantation**
**Cordova Tennessee(US)**

(74) Representative: **Lewin, John Harvey et al**
**ELKINGTON AND FIFE Beacon House 113
Kingsway**
**London WC2B 6PP(GB)**

(54) Methods of making implantable, inflatable silicone reservoirs.

(57) A method of making implantable, inflatable silicone reservoirs by a) coating a section of a first layer of uncured silicone elastomer with a masking material selected from the group consisting of fluorosilicone fluids and curable fluorosilicone mixtures leaving the perimeter around the section free of the masking material, b) placing a surface of a second layer of uncured silicone elastomer in contact with the surface of the first layer so that the second layer contacts the perimeter of the first layer, and c) curing the first and second layers while in contact with each other. The method seals the layers at the perimeter and provides a reservoir between the layers enclosed by the sealed perimeter which has the shape of the coated section. The method is especially suitable for making envelopes for tissue expanders and mammary prostheses and for making the balloon portion of silicone catheters.

# METHODS OF MAKING IMPLANTABLE, INFLATABLE SILICONE RESERVOIRS

The present invention relates generally to methods of making fluid-tight, implantable, inflatable silicone reservoirs such as those used in making tissue expanders, mammary prostheses, or balloon catheters.

Subcutaneous tissue expanders have come into wide use because of the variety of plastic surgical procedures that have been developed which either require that tissue be expanded to receive an implant or that a flap of tissue be generated for use on some other part of the body.

One method of making tissue expanders involves coating a mandrel with a fluid elastomeric composition, curing the composition, and, subsequently, removing the cured composition from the mandrel. An envelope made by this method usually has folds when in its deflated state. Although this method produces very satisfactory envelopes, a flat tissue expander envelope which is generally without folds is sometimes desired.

A second way of making tissue expander envelopes is by sealing two layers of vulcanized elastomer together by placing a washer of raw elastomer between them and curing the washer while in pressure contact with the two layers of vulcanized elastomer.

One type of tissue expander envelope is described in EP 0 196,821, as a one-piece molded body within which is a free-floating member of Teflon or other like non-stick fluorocarbon material of a slightly smaller geometrical shape similar to that of the top and bottom silicone layers of the one piece molded body. The non-stick member acts as a valve, keeps the tube linking the injection port and the expander free and clear of silicon during the molding process, and provides for non-sticking surfaces during expansion.

U.S. Patent No. 4,100,627 to Brill discloses a gel filled flexible article comprising a flexible container containing silicone gel prepared from methylphenylvinylsiloxy-endblocked polydimethylsiloxane. U.S. Patent Nos. 4,455,691 and 4,472,226 to Redinger et al., disclose an implantable prosthesis comprising a flexible sac and a silicone gel contained with the sac. The wall of the sac is comprised of at least one continuous layer of silicone elastomer which substantially impedes the migration of the silicone gel from the sac. The silicone elastomer which impedes the migration is disclosed as preferably being a reaction product of dimethylpolysiloxane and either 3,3,3-trifluoropropylpolysiloxane, diphenylpolysiloxane or methylphenylpolysiloxane. Canadian Patent No. 1,199,451 to Larson teaches of a rubber article comprising a flexible silicone rubber container filled with a silicone gel composition, there being an essentially continuous barrier layer of a composition consisting essentially of a fluorine-containing organopolysiloxane situated between the interior of the container and the gel.

Certain fluorosilicon-containing materials are known as release materials for coating a substrate for releasing silicone pressure sensitive adhesives (hereinafter referred to as SPSAs), e.g., as in Keil, U.S. Patent No. 3,050,411, and Olson, U.S. Patent No. 4,472,480. U.S. Patent No. 4,565,714 to Koshar discloses a low energy release liner for SPSAs comprising the hydrosilylation reaction product of an ethylenically unsaturated perfluoropolyether and a compound bearing silicon-bonded hydrogen atoms.

Copending U.S. patent application Serial No. 870,567 to Brown and Stickles, filed on June 4, 1986, discloses curable fluorosilicone materials which are useful as release coatings for aggressively tacky SPSAs. The application mentions that the compositions may be coated on many substrates including polysiloxanes.

Also, in the SPSA art, U.S. Patent No. 4,039,707 to O'Malley teaches of a SPSA comprising the intercondensation product of a mixture containing an organopolysiloxane resin and an alkylarylpolysiloxane gum and a releasable layer comprising a cured reactive terminal group containing dimethylpolysiloxane fluid-coated sheet on the SPSA.

U.S. Patent No. 4,465,805 to Blizzard et al. discloses curable fluorosilicone coatings which are suitable for coating substrates, such as silicone rubber, and are suitable for use in making release paper.

Other patents cite the use of fluorine-containing silicon-containing materials as general mold release agents, e.g., U.S. Patent No. 3,188,336 to Haszeldine. U.S. Patent No. 3,450,738 to Biochi discloses fluoroorganosilicon compounds of which solutions are useful as mold release agents for plastics. U.S. Patent No. 4,308,212 to Takamizawa et al. discloses fluorine-containing organosilane compounds which are useful for imparting releasability to the surface of shaped articles of synthetic resins, including silicone resins.

Several patents relating to non-silicone catheters disclose ways of partitioning the balloon portion of the catheter from the tube. For example, U.S. Patent Nos. 3,292,627, 3,304,353, and 3,452,756 to Harautuneian and Great Britain Patent No. 1,234,037 to Steer et al. disclose applying a coating of water-soluble polymers between the balloon and the tube of the catheter to effect their separation. In addition, U.S. Patent No. 3,452,756 also mentions that balloon catheters have been made by treating an area of the tube with bromine to cure the rubber and then dipping the tube in a latex to form the rubber balloon which bonded to the rubber tube beyond the ends of the bromine-treated zone. U.S. Patent No. 3,544,668 to Dereniuk discloses a way of keeping the balloon and tube separated by applying a coating of a gel, e.g. methylcellulose, between the balloon and the tube which is later decomposed by drying. U.S. Patent No. 3,528,869, also to Dereniuk, discloses coating the portion of the shaft that is to be covered by the balloon with a release agent, such as Baymal or Silica.

Of the patents which relate to silicone catheters, U.S. Patent Nos. 3,926,705 and 3,983,879 to Todd disclose a catheter having an outer covering, a portion of which is inflatable to form a bubble, and a layer of thermoplastic material applied around the catheter in the region underlying the bubble wherein the themoplastic layer is composed of a material to which the outer covering does not adhere. Todd discloses that when the catheter is silicone, suitable materials for the themoplastic layer are polyethylene or polypropylene tape. U.S. Patent No. 4,536,179 to Anderson et al. discloses a catheter having a silicone tube and a silicone sleeve in face-to-face contact which are provided with a thin film coating of a glow discharge plasma polymerized fluorocarbon between them to prevent adhesion of the contacting surfaces.

U.S. Patent No. 4,413,359 to Akiyama discloses sandwiching a fluororubber (e.g. vinylidene fluoride-hexafluoropropylene-based fluororubber) membrane between two silicone rubber layers to form a laminate membrane.

Several sources cite materials which are useful as mold release agents for silicone elastomers. For example, U.S. Patent No. 3,231,542 to Eisinger et al. teaches that the sticking of silicone elastomers to a mold can be eliminated through the use of conventional mold release agents, such as a dimethylpolysiloxane oil, a diethylpolysiloxane oil or a dimethylpolysiloxane oil modified with phenylmethylsiloxy units or (beta-phenylethyl) methylsiloxy units. The books, Silicones and Their Uses by Rob Roy MacGregor (New York: McGraw Hill Book Co. Inc. 1954) and the Handbook of Silicone Rubber Fabrication by Wilfred Lynch (New York: Van Nostrand Reinhold Co., 1978) cite that solutions of synthetic detergents are useful as release agents for silicone rubber. Lynch also teaches that "[s]ilicone mold releases are not recommended for use with silicone rubbers or resins"; and MacGregor teaches that "[t]he silicone mold-release agents that are commonly used in the molding of organic rubbers are not effective with the silicone rubbers." In the product bulletin 51-437B dated June, 1983, for SILASTIC® 382 Medical Grade Elastomer, a silicone elastomer product which was available from the Dow Corning Corporation, Midland, MI, suitable release agents are disclosed as being "aqueous or alcoholic solutions of detergents, or solvent solutions of waxes, mineral oil, petroleum jelly, or similar substances."

In the book, Compounding Ingredients for Rubber edited by the editors of Rubber World - (The Cuneo Press of New England, 1961), several materials which are used in general rubber compounding are disclosed as being useful for keeping raw or cured rubber stock from adhering together when piled.

According to an abstract of the patent, Japanese Patent application OPI 149962/84 discloses a releasable masking composition prepared by dispersing tetrafluoroethylene telomer in organic solvents. The composition is said to have no adherence to paint. Simultaneous use of silicon type water repellent or fluorine type water and oil repellent, mold release agent or surfactant (e.g., dimethylpolysiloxane, polyfluoroalkyl group containing (meth)acrylate copolymer) facilitates release.

In view of the available methods of making implantable reservoirs, there remains a need for a method of making implantable reservoirs which is easy, requires a minimum of materials, can produce relatively transparent reservoirs, produces flexible reservoirs, can produce reservoirs having unusual, complex shapes, and which method does not result in reservoirs with physical or chemical characteristics which are adversely different from the current implantable reservoirs.

The invention disclosed herein provides a method for making an implantable, inflatable reservoir comprising the steps of a) coating a section of a surface of a first at least partially uncured silicone elastomer layer with a masking material selected from the group consisting of silicone materials which are not readily compatible with the silicone elastomer, such as, a non-curable fluorosilicone composition or a curable fluorosilicone mixture, leaving a perimeter around the section which is substantially free of said masking material, b) placing a surface of a second at least partially uncured silicone elastomer layer in contact with said surface of said first layer so that said second layer contacts substantially the entire length of said perimeter, and c) curing said coated first layer and said second layer while in contact, thereby sealing the layers at the perimeter and providing a reservoir between said layers enclosed by said sealed perimeter and having the shape of said coated section. When the masking material is curable, the method can optionally have the additional step of curing the coated masking material after step (a) and before step (b).

The method is especially suitable for making a low-profile implantable envelope useful for tissue expansion or a mammary prosthesis or for making the balloon portion of a catheter.

Briefly, the invention provides a method for making an implantable silicone reservoir by applying masking material between two layers of silicone elastomer in the area where adhesion is undesirable leaving a perimeter free of the masking material, then, subsequently curing the two layers of silicone elastomer together at their perimeter whereby the layers remain non-adhered in the coated area.

Several types of silicone elastomer are suitable for the invention as long as the silicone elastomers are uncured ("raw") or only partially cured when the masking material is applied. Suitable silicone elastomers for the invention are, e.g., those which cure via $\equiv$SiH to $CH_2 = CHSi\equiv$ addition, in the presence of a catalyst, such as a platinum catalyst, and those which cure by free radical crosslinking, e.g. peroxide-curing elastomer compositions. If the masking material is to be cured, the curing system of the masking material should not interfere with the curing system of the elastomer system and vice versa. For example, elastomer systems based on $\equiv$SiH to $CH_2 = CHSi\equiv$ addition will work in the invention with the curable fluorosilicone materials further discussed below. If using the uncuring masking material, more types of elastomer systems are usable, since there is no concern of inactivating the cure of the masking material and less danger of interfering with the cure of the elastomer system. For example, condensation curable compositions containing siloxanes having $\equiv$SiOH radicals and crosslinkers having $\equiv$SiOR radicals, could be used with the non-curable masking material discussed below. When making low-profile implantable envelopes using the invention, it is preferred that silicone elastomers which cure by $\equiv$SiH to $CH_2CHSi\equiv$ addition be used. When making implantable reservoirs using the invention, the thickness, size, and shape of the elastomer layers will vary with the desired resulting reservoir.

The masking material is a silicone material which is generally non-compatible with the silicone elastomer used. The masking material can be, for example, a non-curable fluorosilicone composition, a curable fluorosilicone composition or the masking material can be a phenylsiloxane composition. Preferably, the silicone material is a curable fluorosilicone composition or a non-curable fluorosilicone composition, and most preferably, the silicone material is a non-curable fluorosilicone composition.

Although the following describes some very specific fluorosilicone materials, it is believed that the invention is not limited to using these particular materials but that other fluorosilicone materials may be used also. Suitable fluorosilicone compositions comprise a fluorosilicone polymer containing at least 2 mol percent, based on the total number of siloxane units in the fluorosilicone polymer, of fluorinated siloxane units, any remaining siloxane units in the polymer being non-fluorinated siloxane units; said fluorinated siloxane units having the formula $(RQ)(R')_a(Z)_bSiO_{(3-a-b)/2}$ and said non-fluorinated siloxane units having the formula $(R')_c(Z)_dSiO_{(4-c-d)/2}$ where, in said fluorinated and non-fluorinated siloxane units, R denotes a perfluoroalkyl radical having less than 9 carbon atoms, Q denotes a divalent hydrocarbon, hydrocarbon ether or hydrocarbon thioether radical linking the R radical to a silicon atom through at least 2 carbon atoms, $R'$ denotes a silicon-bonded, monovalent hydrocarbon radical free of aliphatic unsaturation, Z denotes a silicon-bonded curing radical selected from the group consisting of hydrogen, hydroxyl, and alkenyl, $a = 0$ to 2, $b = 0$ to 2, $a + b = 0$ to 2, $c = 0$ to 3, $d = 0$ to 3 and $c + d = 0$ to 3. Suitable curable fluorosilicone compositions comprise a curable mixture consisting essentially of (A) the fluorosilicone polymer described immediately above where the polymer also contains an average of at least two silicone-bonded curing radicals per molecule and (B) an effective amount of a curing agent for the fluorosilicone polymer.

The fluorosilicone polymer (Component A) described above is an organopolysiloxane consisting essentially of fluorinated siloxane units, optionally, non-fluorinated siloxane units, and, optionally for the non-curable compositions, but required for the curable compositions, silicon-bonded curing radicals.

The silicon-bonded curing radicals are selected from the group consisting of hydrogen atoms, hydroxyl radicals and alkenyl radicals, examples of the latter being vinyl, allyl, butenyl, pentenyl, hexenyl, octenyl and decenyl. Preferably, the aliphatic unsaturation in the alkenyl curing radicals is in the terminal, i.e. omega position.

By fluorinated siloxane units, it is meant siloxane polymer units that bear a perfluoroalkyl radical suitably bonded to a silicon atom. The fluorinated siloxane units have the formula $(RQ)(R')_a(Z)_bSiO_{(3-a-b)/2}$, general examples of which include chain-terminating siloxane units having the formula $(RQ)(R')_a(Z)_bSiO_{1/2}$, where the sum of $a + b$ is 2, such as $(RQ)(R')_2SiO_{1/2}$, $(RQ)(Z)_2SiO_{1/2}$ and $(RQ)(R')(Z)SiO_{1/2}$, chain-extending siloxane units having the formulae $(RQ)(R')SiO_{2/2}$ and $(RQ)(Z)SiO_{2/2}$ and chain-branching siloxane units having the formula $(RQ)SiO_{3/2}$.

The non-fluorinated siloxane units, if present, have the formula $(R')_c(Z)_d SiO_{(4-c-d)/2}$, general examples of which include chain-terminating siloxane units having the formula $(R')_c(Z)_d SiO_{1/2}$ where the sum of $c+d$ is 3, such as $(R')_3 SiO_{1/2}$, $(R')_2(Z)SiO_{1/2}$, $(R')(Z)_2 SiO_{1/2}$ and $(Z)_3 SiO_{1/2}$; chain-extending siloxane units having the above formula where the sum of $c+d$ is 2, such as $(R')_2 SiO_{2/2}$, $(R')(Z)SiO_{2/2}$ and $(Z)_2 SiO_{2/2}$ and chain-branching siloxane units having the above formula where the sum of $c+d$ is 1 or 0, such as $(R')SiO_{3/2}$, $(Z)SiO_{3/2}$ and $SiO_{4/2}$.

Although the fluorosilicone polymer can have any viscosity up to several million centistokes, it is believed necessary that the polymer not be a nonfluid, such as a gel or a solid. Therefore, said chain-branching siloxane units, if present, should be present in only minor amounts.

It is preferred that the fluorosilicone polymer be made up of only chain-extending and chain-terminating siloxane units selected from the group consisting of $YMe_2 SiO_{1/2}$, $RQMeYSiO_{1/2}$, $MeYSiO_{2/2}$ and $RQYSiO_{2/2}$ siloxane units; wherein Y denotes Me or A, A denotes an omega-alkenyl radical and Me denotes the methyl radical. The presence of other chain-terminating and/or chain-extending siloxane units would lead to polymers that contain silicon atoms that bear a plurality of curing radicals, and would be expected to present synthesis, as well as curing, problems.

Specific examples of said selected siloxane units include, but are not limited to, $Me_3 SiO_{1/2}$, $Me_2 ViSiO_{1/2}$, $RQMe_2 SiO_{1/2}$, $RQMeViSiO_{1/2}$, $Me_2 SiO_{2/2}$, $MeViSiO_{2/2}$, $RQMeSiO_{2/2}$ and $RQViSiO_{2/2}$, where R is, for example, perfluorobutyl.

General examples of preferred fluorosilicone polymers include, but are not limited to, the following:

$YMe_2 SiO(MeYSiO)_m(RQYSiO)_n SiMe_2 Y$,

$RQMeYSiO(MeYSiO)_m(RQYSiO)_n SiMeYRQ$,

$Me_3 SiO(Me_2 SiO)_{0.95m}(MeViSiO)_{0.05m}(RQMeSiO)_n SiMe_3$,

$ViMe_2 SiO(Me_2 SiO)_m(RQMeSiO)_n SiMe_2 Vi$,

$ViMeRQSiO(RQMeSiO)_n SiMeRQVi$,

$Me_2 RQSiO(RQMeSiO)_{0.95n}(RQViSiO)_{0.05n}$ and

$Me_3 SiO(Me_2 SiO)_m(RQMeSiO)_{0.90n}(RQViSiO)_{0.10n} SiMe_3$,

wherein the viscosity of the polymer ranges from that of a freely flowing liquid to a slowly flowing gum and m and n have values of from zero to 10,000 and more.

It is preferred that the fluorosilicone polymer have a linear structure and be represented by the formula

$YMe_2 SiO(Me_2 SiO)_x[RCH_2 CH_2 Si(Me)O]_y(MeASiO)_z SiMe_2 Y$

wherein the values of x and y are each greater than zero, and x, y, and z are such that the fluorosilicone polymer contains at least 5 mol percent fluorinated siloxane units. In the case of curable compositions, it is preferred that the fluorosilicone polymer additionally have in-the-chain curing radicals where z is greater than 0 and x, y, and z are such that the fluorosilicone polymer contains from 1 to 10 mol percent alkenyl-containing siloxane units and the balance dimethylsiloxane units and the curing agent comprises a mixture of a platinum-containing hydrosilylation catalyst and a methylhydrogenpolysiloxane having the formula $Me_3 SiO(MeHSiO)_e SiMe_3$ wherein e has a value of from 30 to 70.

Even more preferred are compositions wherein the fluorosilicone polymer has a linear structure as represented by the formula noted immediately above wherein the values of x and y are each greater than zero and are such that the fluorosilicone polymer contains from 20 to 50 mol percent fluorinated siloxane units and has a viscosity of from 100 to 1000 centistokes at 25° C. When the composition is curable, more preferred compositions are those where z is greater than 0 and x, y, and z are such that the fluorosilicone polymer contains from 3 to 7 mol percent vinyl-containing siloxane units and the balance dimethylsiloxane units and the curing agent comprises a platinum-containing hydrosilylation catalyst and a methylhydrogenpolysiloxane having the formula $Me_3 SiO(MeHSiO)_e SiMe_3$ wherein e has a value of from 30 to 70.

The terminal Y radicals can be methyl or alkenyl, such as vinyl, without significantly altering the masking ability. However, for curable compositions it may be desirable that the terminal Y radicals be alkenyl under moderate curing conditions, such as low curing temperatures, short curing times or attenuated curing catalyst activity.

In the above formulae for the fluorosilicone polymer and its siloxane units, R denotes a perfluoroalkyl radical having from less than 9 carbon atoms, over the complete range of from 2 to 100 mol% fluorinated siloxane units. The R radicals can be identical or different and can have a normal or a branched structure. Examples thereof include $CF_3-$, $C_2 F_5-$, $C_3 F_7-$, $C_4 F_9-$, such as $CF_3 CF_2 CF_2 CF_2-$, $(CF_3)_2 CFCF_2-$, $(CF_3)_3 C-$ and $CF_3 CF_2(CF_3)CF-$; $C_5 F_{11}-$, such as $CF_3 CF_2 CF_2 CF_2 CF_2-$; $C_6 F_{13}-$, such as $CF_3(CF_2)_4 CF_2-$; $C_7 F_{14}-$, such as $CF_3(CF_2 CF_2)_3-$; and $C_8 F_{17}-$.

Preferably, R is a perfluoroalkyl radical having from 4 to 8 carbons atoms and the fluorosilicone polymer contains from 2 to 100 mol percent fluorinated siloxane units, or R is a perfluoroalkyl radical having from 2 to 3 carbons atoms when the fluorosilicone polymer contains less than 90 mol percent fluorinated siloxane units, or R is a perfluoroalkyl radical having 1 carbon atom when the fluorosilicone polymer contains from 7 to 10 mol percent fluorinated siloxane units.

It is reasonable, and within the scope and spirit of the present invention, that R can be $C_9F_{19}$-, $C_{10}F_{21}$-, and larger. However, it is clear that polymers containing perfluoroalkyl radicals containing 1 to 8 carbon atoms, depending upon the amount of fluorinated siloxane units in the fluorosilicone polymer, provide excellent results and that the use of larger perfluoroalkyl radicals would only provide incremental improvements at higher cost.

Each perfluoroalkyl radical is bonded to a silicon atom by way of Q, a divalent spacing radical containing carbon, hydrogen and, optionally, oxygen and/or sulfur atoms which are present as ether and thioether linkages, respectively. The sulfur and oxygen atoms, if present, must be bonded to only carbon atoms.

Each Q radical can have any structure containing the elements listed; however, each is preferably an alkylene radical having a normal or branched structure. Examples of suitable alkylene radicals include $-CH_2CH_2-$, $-CH_2CH_2CH_2-$, $-CH_2(CH_3)CH_2-$, $-(CH_2CH_2)_2-$, $-CH_2(CH_3)CH_2CH_2-$ and $-CH(CH_3)-CH_2-$.

Each fluorinated radical, RQ, preferably has the formula $RCH_2CH_2-$. The fluorosilicone polymers preferably contain fluorinated siloxane units delineated above whose RQ radicals have the structure $CF_3CF_2CF_2CF_2Q-$, and most preferably $CF_3CF_2CF_2CF_2CH_2CH_2-$.

In the above formulae for the fluorosilicone polymer and its siloxane units, R′ denotes a silicon-bonded monovalent hydrocarbon radical, preferably having from 1 to 6 carbon atoms, and containing no aliphatic unsaturation. The R′ radicals can be identical or different, as desired. Examples of suitable R′ radicals include alkyl radicals, such as methyl, ethyl, propyl, isopropyl, butyl, hexyl, 2-ethyl-hexyl, octyl, isooctyl and decyl; aryl, such as phenyl, tolyl, benzyl, beta-phenylethyl and styryl. To provide optimum masking, it is believed necessary that at least 90 percent, and preferably all, of the R′ radicals in the fluorosilicone polymer be methyl radicals.

In the above formulae for the fluorosilicone polymer and its siloxane units, Z denotes a silicon-bonded curing radical selected from the group consisting of hydrogen, hydroxyl and alkenyl, as delineated above.

In the above formulae for the siloxane units, the values of a, b, c and d denote integers, the values of which are as delineated.

The values of m, n, x, y and z for the linear fluorosilicone polymer denote average values, as is well known in the art, and are such that the polymer contains the requisite amount of alkenyl-containing siloxane units in the case of curable compositions and fluorinated siloxane units and has the desired viscosity at 25°C. The values of m, n, m + n, x, y, z and x + y + z thus will vary greatly, depending on the fluorinated siloxane unit content, the structure of the fluorinated radicals and the viscosity of the polymer.

While the values of x and y can be as small as one and the value of z can be as small as 0, the values of x and y can range to 10,000 and more and the value of z typically is zero or limited to a fraction, such as from 1/100 to 2/10, of the sum of x + y + z.

When formulated with an effective amount of a suitable curing agent the curable fluorosilicone polymers containing an average of at least two curing radicals per molecule can be cured. Suitable curing agents, Component (B), for Component (A) comprise a crosslinking agent, examples of which include, but are not limited to, aliphatically unsaturated compounds to react with silicon-bonded hydrogen curing radicals, and organohydrogen silicon compounds bearing a plurality of silicon-bonded hydrogen atoms to react with silicon-bonded alkenyl curing radicals and/or silicon-bonded hydroxy curing radicals. Additionally, the curing agent typically comprises a curing catalyst to accelerate the reaction of the curing radicals with the crosslinking agent, particularly at elevated temperature.

Examples of aliphatically unsaturated cross linking agents include organosilicon compounds such as silanes and cyclic, linear and resinous siloxanes which bear a plurality of silicon-bonded alkenyl radicals.

Examples of organohydrogen silicon cross linking agents include any organosilicon compound which bears a plurality of silicon-bonded hydrogen atoms, such as cyclic, linear and resinous siloxanes, such as methylhydrogencyclopolysiloxanes having the unit formula $MeHSiO_{2/2}$; linear methylhydrogenpolysiloxanes having the formulae $Me_3SiO(MeHSiO)_i(Me_2SiO)_jSiMe_3$ and $HMe_2SiO(MeHSiO)_i(Me_2SiO)_jSiMe_2H$ where i and j have values of zero or more; branched siloxanes such as $(HMe_2SiO)_4Si$ and the fluorosilicone cros-slinkers disclosed by Holbrook in U.S. Patent No. 3,344,160; and the resinous crosslinkers disclosed by Blizzard et al. in U.S. Patent No. 4,310,678, said patents further teach the scope and synthesis of said fluorosilicone crosslinkers and said resinous crosslinkers.

Examples of suitable, well known curing catalysts include, but are not limited to, organoperoxides, platinum-group metals and their compounds, and tin and lead salts of carboxylic acids. such as stannous octoate and dibutyltin diacetate.

The curable compositions usable in this invention preferably comprise a curing agent which comprises a platinum-containing hydrosilylation catalyst and a methylhydrogenpolysiloxane having the formula $Me_3SiO(MeHSiO)_eSiMe_3$ wherein e has a value of from 30 to 70. A particularly useful platinum-containing catalyst for the curable compositions is the chloroplatinic acid-vinylsiloxane complex disclosed by Willing in U.S. Patent No. 3,419,593. However, the platinum-containing catalyst can be any of the well known materials that are effective for catalyzing the hydrosilylation reaction of silicon-bonded hydrogen atoms with silicon-bonded vinyl radicals.

The amount of curing agent to be used in the curable compositions is not normally critical, it only being necessary to have an effective amount thereof to cure the composition to the desired amount. Typically, an effective amount of a curing agent will contain a sufficient amount of crosslinking agent to provide one or more crosslinking radicals for every curing radical in the fluorosilicone polymer. When the curing agent comprises a methylhydrogenpolysiloxane, it is preferred that a sufficient amount thereof would provide from 1 to 10, preferably from 1 to 4, silicon-bonded hydrogen atoms for every curing radical in the fluorosilicone polymer.

It is usually desirable to use a sufficient amount of a curing catalyst in the curable compositions to provide a rapid cure rate. The exact amount of said catalyst will depend on the particular catalyst that is used and is not easily predicted. However, for chloroplatinic acid and its complexes, an amount sufficient to provide from 10 to 500 parts by weight of platinum for every one million parts by weight of the fluorosilicone polymer is usually sufficient. Within this range, routine experimentation can be used to determine the optimum amount of catalyst needed for any particular cure time.

The compositions can further comprise various amounts of optional components that will not adversely limit the use of the composition as a masking material for silicone elastomers. Examples thereof include reactive components, such as catalyst activity attenuators to inhibit the activity of the catalyst, if used, at room temperature and unreactive components such as diluents to decrease the viscosity of the composition.

Preferred diluents include halogenated solvents, such as chlorofluorocarbons; esters, such as ethyl acetate; ketones such as methylisobutyl ketone; and ethers, such as dibutyl ether. Preferred catalyst activity attenuators include methylvinylcyclosiloxanes; esters of unsaturated alcohols and/or unsaturated acids, such as diallyl maleate and bis(2-methoxyisopropyl) maleate; acetylenic compounds, such as methylbutynol; and eneynes, such as ethynylcyclohexene. The reader is referred to, for example, the disclosures of U.S.-. Patent Nos. 3,445,420; 4,256,870; 4,465,818 and 4,562,096, to further illustrate the optional attenuator component of the compositions.

The alkenyl-containing copolymers discussed above and disclosed in copending U.S. patent application Serial No. 870,567 are believed to be novel in the fluorosilicone art.

In some instances, it may be preferred to use curable fluorosilicone materials rather than non-curable fluorosilicone compositions in the invention. For example, when it is desired to minimize the amount of flow of the material on the elastomer before curing the elastomer, it may be preferred to cure the masking material in place. Additionally, when curable fluorosilicone materials are used as the masking material and the envelope is to be filled with a silicone gel, the envelope may have the additional advantage of being more "bleed resistant", i.e. more resistant to migration of silicone gel through the envelope wall.

Although the curable compositions discussed above are quite suitable for the method of this invention, non-curable fluorosilicone compositions were found to be preferable to the curable compositions. The curable materials have the potential for premature gellation, and, for this reason alone, it may be more desirable to use non-curable fluorosilicone materials as the masking material.

The fluorosilicone polymers can be prepared by any of several methods disclosed in the art. For example, the hydroxy-terminated polymers can be prepared by the method of Johannson, U.S. Patent No. 3,002,951 or Brown, U.S. Patent No. 3,179,619. The organo-terminated polymers can be prepared by the method of Pierce et al., U.S. Patent No. 2,961,425. In addition, the fluorosilicone polymers may be prepared using the preferred method as disclosed in copending U.S. Serial No. 870,567, filed on June 4, 1986, and stated generally below.

The vinyl-containing copolymers of the general formula

$YMe_2SiO(Me_2SiO)_x[RCH_2CH_2Si(Me)O]_y(MeASiO)_zSiMe_2Y$,

wherein the values of x, y and z are each greater than zero, are preferably prepared by the method as disclosed as the preferred method in copending U.S. patent application Serial No. 870,567.

Copending U.S. patent application Serial No. 870,567 describes the process for preparing fluorosilicone copolymers as comprising the steps of (I) hydrolyzing a mixture comprising one or more fluorinated silanes having the formula $(RQ)(R'')_fSiX_{(3-f)}$ and one or more non-fluorinated silanes having the formula $R''_gSiX_{(4-g)}$ where, in said fluorinated and non-fluorinated silanes, R denotes a perfluoroalkyl radical having from 1 to 8 carbon atoms, Q denotes a divalent hydrocarbon, hydrocarbon ether or hydrocarbon thioether radical linking the R radical to the silicon atom through at least 2 carbon atoms, $R''$ denotes a silicon-bonded radical selected from the group consisting of monovalent hydrocarbon radicals and hydrogen atoms, f has a value of 0, 1 or 2, g has a value of 0, 1, 2, or 3 and X denotes a silicon-bonded hydrolyzable radical, (II) mixing with the hydrolyzed mixture obtained in (I) an organopolysiloxane having the average unit formula $R''_h SiO_{(4-h)/2}$ wherein $R''$ has the meaning noted above and h has an average value of from 1 to 3 and (III) contacting the mixture of (II) with an effective amount of a siloxane-equilibrating catalyst for a period of time sufficient to form the desired fluorosilicone polymer.

In step (I), a cohydrolyzate of a mixture of fluorinated and non-fluorinated silanes is first prepared which has greater compatibility with polydimethylsiloxanes than does the hydrolyzate of the fluorinated silanes alone. It has been found that cohydrolyzates having as much as 90 percent fluorinated siloxane units have this improved compatibility with polydimethylsiloxanes. However, it is desirable to incorporate as much non-fluorinated silane into the hydrolyzate as possible, based on the composition of the polymer to be prepared and the compatibility of the silanes. Thus, for the purpose of preparing the fluorosilicone polymers which contain up to 50 mol percent fluorinated siloxane units, it is preferred to prepare a cohydrolyzate having up to two fluorinated siloxane units for every one non-fluorinated siloxane unit and then to introduce any additional non-fluorinated siloxane units, into the cohydrolyzate in the second step of this process.

The silanes that are mixed and cohydrolyzed in (I) bear at least one hydrolyzable radical (X) per molecule. Although the hydrolyzable radicals are preferably chlorine atoms, it is believed that they can also be any other halogen atom, an alkoxy radical such as methoxy-or ethoxy, an acyloxy radical such as acetoxy or an amino radical such as $NH_2$ or NH. Examples of suitable fluorinated silanes include RQMeYSiX and $RQYSiX_2$, such as $RQ(Me)SiCl_2$, $RQ(Vi)SiCl_2$, $RQ(Me)_2SiCl$ and RQ-(Me)(Vi)SiCl. Examples of suitable non-fluorinated siloxane units include $YMe_2SiX$ and $MeYSiX_2$, such as $Me_2SiCl_2$, $MeViSiCl_2$, $Vi(Me)_2SiCl$ and $Me_3SiCl$.

The silanes are preferably dissolved in a water-insoluble solvent such as a dialkylether and the resulting solution added to water with vigorous agitation. If halosilanes are not used, it is preferred that the water be made acidic with a mineral acid such as hydrochloric acid.

The resulting hydrolyzate is then freed of any solvent and mixed with an organopolysiloxane having the formula $R''_h SiO_{(4-h)/2}$, examples of which include cyclic siloxanes having the formulae $(Me_2SiO)_i$ and $(MeASiO)_i$, wherein i has a value of at least 3, such as $[(Me)_2SiO]_{3-10}$ and $[(Me)(Vi)$-

$SiO]_{3-10}$; and linear siloxanes having the formula $YMe_2SiO(Me_2SiO)_j(MeViSiO)_k SiMe_2Y$, wherein j and k have values of zero or more, such as $Me_3SiO(Me_2SiO)_{0-10}(MeViSiO)_{0-10}SiMe_3$ and $ViMe_2 SiO(Me_2SiO)_{0-10}(MeViSiO)_{0-10}SiMe_2Vi$.

The mixture of hydrolyzate and organopolysiloxane is brought into contact with a siloxane-equilibrating catalyst such as an acidic catalyst such as sulfuric acid-treated clays or ion-exchange resins, fluoroalkanesulfonic acids, perfluoroalkanesulfonic acids or mineral acids such as hydrochloric acid or sulfuric acid; or a basic catalyst such as alkali metal hydroxides, alkali metal silanolates or tetraalkyl ammonium or phosphonium hydroxides or silanolates. The temperature of the mixture that is contacted with the acid is not critical since copolymer formation will occur at room temperature if a sufficient amount of time is allowed for the reaction to occur. However, it is preferred to accelerate this reaction by heating the reaction mixture, for example to 100 to 200°C.

After the fluorosilicone polymer has been formed, as indicated by no further change in the viscosity of the reaction mixture, the catalyst is preferably deactivated, such as by neutralization; although this step is not necessary. It is also desirable, but not necessary, to remove volatile materials from the fluorosilicone polymer before it is used for this invention.

As mentioned, the masking materials can be applied to the elastomer surface in a solvent free state or as a dispersion. For higher viscosity fluorosilicones, it is preferred that the silicones be applied in dispersion so that the viscosity is low enough to apply a substantially uniform layer by the desired technique but not so low that the dispersion flows to a large extent on the elastomer before curing of the elastomer.

The compositions can be applied by any suitable manner such as by brushing, spreading, spraying, rolling, swabbing, gravure, kiss roll, air knife or doctor blade.

In the method of this invention, it is preferred to uniformly coat the elastomer in the desired area at least a monomolecular layer thick, preferably a multimolecular layer thick, so long as the layer is not so thick that it flows undesirably. The masking material is applied to the section of the silicone elastomer where non-adhesion to the other elastomer layer is desired, leaving substantially the entire length of the perimeter uncoated. The entire perimeter does not have to be left uncoated, if there is a portion of the perimeter where non-adherence is desired. For example, it may be desirable to leave an opening in the reservoir at the perimeter to later attach an inlet tube in the case of a tissue expander envelope. Therefore, substantially all of the perimeter is free of masking material, but not necessarily all of it. If masking material accidentally flows onto an undesired portion of the elastomer, it may be possible to remove the masking material with a suitable solvent.

The coated curable compositions may be cured onto the first layer of elastomer before contacting the second layer of elastomer to the first or the coated curable composition may remain uncured until the two layers of elastomer are cured together while in contact with each other. Generally, the curable fluorosilicone materials can cure at room temperature, but, typically, the cure is accelerated with heat. If it is desired to cure the coated curable composition before contacting the second layer of elastomer to the first, a suitable low temperature for curing would be about 86° C.; a suitable time for curing would be until the fluorosilicone material is substantially tack-free. Higher temperatures would be suitable, so long as the conditions do not prematurely cure the elastomer layer also. Temperatures above 170° C. would probably be unnecessary as thin layers of the curable fluorosilicone materials cure very quickly at this temperature.

Preferably, after coating the first layer of uncured silicone elastomer, the second layer of uncured silicone elastomer is placed in contact with the first layer. It is best to apply pressure to the silicone layers where bonding is desired between the two elastomer layers, e.g., at their perimeter. One could also practice the invention by first placing the two layers of elastomer in contact, then by injection, apply the masking material between the layers. For example, two sheets of elastomer could be stacked together, pressure applied around the perimeter, and masking material injected between the layers using a syringe and needle. The stacked layers could then be rotated to allow the masking material to spread.

The contacting silicone elastomer layers are then cured, preferably under pressure and elevated temperatures. With silicone elastomers based on $\equiv$SiH to $CH_2 = CHSi\equiv$ addition, it was found that a pressure of 3 tons and a temperature of 300° F. was very suitable. Upon curing the elastomer layers together while in contact, a composite is formed having gaps within it where the masking material is present and the elastomers did not bond together. For example, in the case of making envelopes for tissue expanders, two sheets of silicone elastomer are cured while in contact with one another with masking material between them except at their perimeter. Upon curing, the elastomer sheets cure or bond together at the perimeter and not at the inner portion of their facing surfaces, thus, forming a fluid-tight reservoir.

Although the masking has been described as being placed on one side of an elastomer layer, the masking material can be placed on both sides of an elastomer layer if masking is desired on both sides, especially when more than two layers of elastomer are employed.

The method of the invention is also useful for forming reservoirs having unusual and/or complex shapes that are not easily constructed using the methods of the prior art. For example, a doughnut-shaped reservoir having a hole in the middle can be made with this method but is not easily made using a mandrel.

In the general sense, this invention provides a method for blocking the adhesion between layers of silicone elastomer during cure by applying a silicone material which is generally not compatible with the silicone elastomer, such as the fluorosilicone materials described above, between the layers of silicone elastomer before curing. In the general sense, the method comprises the steps of a) coating a section of a surface of a first at least partially uncured silicone elastomer body with the masking material, b) placing a second at least partially uncured silicone elastomer body in contact with the surface of the first body, and c) curing the first and second bodies while in contact, whereby the bodies remain unadhered to each other in the area of the coated section, and any uncoated sections of the silicone bodies in contact with each other during cure will bond together.

The variations discussed above and other variations of the present invention may be made which fall within the scope of the appended claims even though such variations were not specifically discussed above.

The following examples are disclosed to further teach how to practice the present invention and should not be taken as limiting the invention, which is properly delineated by the appended claims.

In the following examples, Me and Vi denote methyl and vinyl, respectively, temperatures are in degrees Celsius and all parts are by weight, unless otherwise specified.

## Example 1

This example illustrates the method of making tissue expander envelopes using the present invention and employing curable fluorosilicone compositions.

A mixture of 20.3 parts of heptane, 8.4 parts of $Me_2SiCl_2$ and 23.6 parts of $(CF_3CF_2CF_2CF_2CH_2CH_2)(CH_3)SiCl_2$ was slowly added to 47.7 parts of stirred water. An exotherm to 55° resulted. The resulting hydrolysis mixture was stirred for 45 minutes and was then allowed to stand until a two-phase system resulted. The aqueous phase was separated and discarded. The organic phase was washed once with 50 parts of 10% aqueous NaCl, after which the organic phase was found to be neutral with litmus paper. The heptane was then removed from the organic phase at a pressure of 150 mm Hg and 40 to 55° and the residue was freed of additional volatile material by heating to 80° at 25 mm Hg vacuum. The cohydrolyzate residue was a copolymer of 50 mols of $(CF_3CF_2CF_2CF_2CH_2CH_2)(CH_3)Si_{2/2}$ siloxane units and 50 mols of $(CH_3)_2SiO_{2/2}$ siloxane units.

A mixture of 32.93 parts of the 50/50 cohydrolyzate, 42.31 parts of $[(Me_2)SiO]_4$, 6.50 parts of $[(Me)(Vi)SiO]_5$, 1.58 parts of $Me_3SiO$-$(Me_2SiO)_{10}SiMe_3$, 16.67 parts of heptane and 0.09 parts of $CF_3SO_3H$ catalyst was heated to 120° and held at that temperature for 5 hours, after which it was cooled, mixed with 0.90 parts of $NaHCO_3$ and 0.90 parts of diatomaceous earth and pressure-filtered. The filtrate was devolatilized at 250 degrees/10 mm Hg for 15 minutes. The resulting mixture, hereinafter referred to as Fluorosilicone Polymer "A", had a viscosity of 500 centistokes at 25° C., about 10 mol % fluorinated siloxane units and about 9 mol % in-the-chain vinyl siloxane units, and believed to have an average chemical formula of $Me_3SiO(Me_2SiO)_{161}(MeViSiO)_{18}$-$(MeRQSiO)_{21}SiMe_3$ where $RQ = -CH_2CH_2(CF_2)_4F$.

Fluorosilicone Composition "A" was prepared by homogeneously mixing a composition consisting of 10 parts of Fluorosilicone Polymer "A", 89.6 parts of trichlorotrifluoroethane, 0.1 parts of methylvinylcyclosiloxane, and 0.3 parts of a complex of divinyltetramethyldisiloxane and $H_2PtCl_6$. 0.8 parts $Me_3SiO(MeHSiO)_{50}SiMe_3$ was added to 100 parts of Fluorosilicone Composition "A" to make Curable Fluorosilicone Composition "A".

A sheet of 0.04" thick unvulcanized silicone elastomer consisting essentially of a vinyl-containing dimethylpolysiloxane gum elastomer base, a dimethylmethyl-hydrogenpolysiloxane cross linker and a platinum catalyst measuring approximately 4 inches wide by 4 inches long was placed onto a compression plate. A template for covering about 1 4 inch of the perimeter of the sheet was placed on the top surface of the sheet. About 2-3 grams of Curable Fluorosilicone Composition "A" was applied, using a foam swab, to the top surface of the sheet in the area exposed by the template, thereby leaving a perimeter of about 1/4 inch wide around the top surface of the sheet free of coating. The template was then removed from the sheet and the coated sheet was placed in an oven heated to about 320° F. for about two minutes to cure the coating.

Another sheet of 0.04" thick unvulcanized silicone elastomer of similar dimensions to the other sheet was placed onto the coated silicone sheet. Manual pressure was applied to the perimeter of the layered sheets insuring good contact between the sheets in the area free of the Curable Fluorosilicone Composition "A". Using a hypodermic needle with a 23 gauge needle, the trapped air was removed from the pocket created between the two layers of sheeting. Then a 0.075" chase was placed around the sheeting composite, a compression plate was placed on top of the sheeting composite and chase. The compression plates, sheeting and chase were placed in a compression press and three tons of pressure was applied at 300° F. for 5 minutes. The compression plates were then removed from the press and the silicone envelope removed from the plates. Using a hypodermic needle with a syringe filled with air, the envelopes were expanded and the two layers of silicone elastomer separated in the area where the Curable Fluorosilicone Composition "A" was applied.

Example 2

This example illustrates the method of making tissue expander envelopes using the present invention employing non-curable fluorosilicone compositions.

A tissue expander envelope was prepared as described in Example 1 except Fluorosilicone Composition "A" was applied on the elastomer sheet rather than Curable Fluorosilicone Composition "A". After the envelope was removed from the compression plates, the envelope was expanded using a hypodermic needle with a syringe filled with air and the two layers of silicone elastomer separated in the area where the Fluorosilicone Composition "A" was applied.

Example 3

This example illustrates the method of making tissue expander envelopes of the present invention employing non-curable fluorosilicone compositions wherein the fluorosilicone polymer is free of curing radicals.

When the procedure as described in Example 1 is followed substituting a fluorosilicone polymer having the average structure of $Me_3SiO(Me_2SiO)$-$_{79}(MeRQSiO)_{21}SiMe_3$ where $RQ = -CH_2CH_2$-$(CF_2)_4F$ for the Curable Fluorosilicone Composition "A", a tissue expander envelope is prepared.

Claims

1. A method for making an implantable, inflatable reservoir comprising the steps of:

a) coating a section of a surface of a first at least partially uncured silicone elastomer layer with a masking material selected from the group consisting of non-curable fluorosilicone compositions and curable fluorosilicone compositions, leaving a perimeter around said section substantially free of said masking material,

b) placing a surface of a second at least partially uncured silicone elastomer layer in contact with said surface of said first layer so that said second layer contacts substantially the complete length of said perimeter, and

c) curing said coated first layer and said second layer while in contact, thereby sealing the layers at the perimeter and providing a reservoir between said layers enclosed by said sealed perimeter and having the shape of - said coated section.

2. The method as claimed in claim 1 wherein the masking material comprises a curable fluorosilicone mixture consisting essentially of

(A) a fluorosilicone polymer containing an average of at least two silicon-bonded curing radicals per molecule selected from the group consisting of hydrogen, hydroxyl and alkenyl and at least 2 mol percent, based on the total number of siloxane units in the fluorosilicone polymer, of fluorinated siloxane units, any remaining siloxane units in the polymer being nonfluorinated siloxane units; said fluorinated siloxane units having the formula
$(RQ)(R')_a(Z)_bSiO_{(3-a-b)/2}$
and said non-fluorinated siloxane units having the formula
$(R')_c(Z)_dSiO_{(4-c-d)/2}$
where, in said fluorinated and non-fluorinated siloxane units, R denotes a perfluoroalkyl radical having less than 9 carbon atoms, Q denotes a divalent hydrocarbon, hydrocarbon ether or hydrocarbon thioether radical linking the R radical to a silicon atom through at least 2 carbon atoms, $R'$ denotes a silicon-bonded, monovalent hydrocarbon radical free of aliphatic unsaturation, Z denotes said silicon-bonded curing radical, $a = 0$ to 2, $b = 0$ to 2, $a + b = 0$ to 2, $c = 0$ to 3, $d = 0$ to 3 and $c + d = 0$ to 3, and

(B) an effective amount of a curing agent for the fluorosilicone polymer.

3. A method as claimed in claim 1 further comprising the step of curing said coated masking material after step (a) and before step (b).

4. A method as claimed in claim 2 further comprising the step of curing said coated masking material after step (a) and before step (b).

5. The method as claimed in claim 1 wherein the masking material comprises a fluorosilicone polymer having at least 2 mol percent, based on the total number of siloxane units in the fluorosilicone polymer, of fluorinated siloxane units, any remaining siloxane units in the polymer being non-fluorinated siloxane units; said fluorinated siloxane units having the formula $(RQ)(R')_a(Z)_bSiO_{(3-a-b)/2}$ and said non-fluorinated siloxane units having the formula $(R')_c(Z)_dSiO_{(4-c-d)/2}$ where, in said fluorinated and non-fluorinated siloxane units, R denotes a perfluoroalkyl radical having less than 9 carbon atoms, Q denotes a divalent hydrocarbon, hydrocarbon ether or hydrocarbon thioether radical linking the R radical to a silicon atom through at least 2 carbon atoms, $R'$ denotes a silicon-bonded, monovalent hydrocarbon radical free of aliphatic unsaturation, Z denotes a silicon-bonded curing radical selected from the group consisting of hydrogen, hydroxyl, and alkenyl, $a = 0$ to 2, $b = 0$ to 2, $a + b = 0$ to 2, $c = 0$ to 3, $d = 0$ to 3, and $c + d = 0$ to 3.

6. A reservoir produced in accordance with the method of claim 1.

7. A reservoir produced in accordance with the method of claim 2.

8. A reservoir produced in accordance with the method of claim 5.